# EUROPÄISCHE PATENTSCHRIFT

(11) **EP 2 700 426 B1**
(45) Veröffentlichungstag und Bekanntmachung des Hinweises auf die Patenterteilung: **04.04.2018**
(21) Anmeldenummer: 13180186.2
(22) Anmeldetag: 13.08.2013
(51) Int. Cl.: A61M 5/34, A61M 5/31

(54) **Spritze mit drehbarer Gewindehülse**
Syrige with a rotatable bush
Seringue avec douille filetée rotative

(30) Priorität: 20.08.2012 DE 102012214718
(43) Veröffentlichungstag der Anmeldung: 26.02.2014
(73) Patentinhaber: Transcoject GmbH, 24539 Neumünster (DE)
(72) Erfinder: Heinz, Jochen, 24220 Flintbek (DE)
(74) Vertreter: Patentanwälte Vollmann & Hemmer

(56) Entgegenhaltungen:
- EP-A1- 1 733 749
- WO-A1-89/09071
- WO-A1-2006/087763
- DE-A1-102009 019 340

## Beschreibung

Die Erfindung betrifft eine Spritze, insbesondere eine medizinische Spritze, mit den im Oberbegriff des Anspruchs 1 angegebenen Merkmalen.

Spritzen bzw. medizinische Spritzen weisen an ihrem Ende in der Regel einen Luer-Anschluss zur Verbindung mit einer Kanüle oder einem anzuschließenden Schlauch oder ähnlichem auf. Um diese Luer-Verbindung zu sichern, sind so genannte Luer-Lock-Anschlüsse bekannt, welche ein den Luer-Konus beabstandet umgebendes Innengewinde aufweisen, das mit dem Gegenanschluss verschraubt wird. Dies kann einerseits durch Drehung des Gegenanschlusses oder aber durch Drehung des Gewindes, d. h. der Gewindehülse des Luer-Lock-Anschlusses erfolgen, wozu die Gewinderhülse drehbar an dem Luer-Konus befestigt ist. Beim Anschluss muss jedoch die Gewindehülse ergriffen werden, um sie zu drehen. Dabei besteht die Gefahr, dass die Finger mit der Spitze des Luer-Konus selber in Kontakt kommen, wodurch Bereiche, welche mit dem Inhalt der Spritze in Kontakt kommen, verunreinigt werden könnten.

EP 1 733 749 A1 offenbart eine Spritze mit einem Luer-Lock-Anschluss mit einer drehbaren Gewindehülse. Dabei ist die Gewindehülse zweiteilig oder aufklappbar ausgebildet, um auf den Konus der Spritzenzylinders aufgesetzt zu werden. Zum Fixieren der Teile der Gewindehülse im geschlossenen Zustand ist eine aufschraubbare Sicherungsmutter vorgesehen. Auch bei dieser Ausgestaltung ist die Gewindehülse relativ klein, so dass die Gefahr besteht, dass die Finger mit der Spitze des Luer-Konus selber in Kontakt kommen, wodurch Bereiche, welche mit dem Inhalt der Spritze in Kontakt kommen, verunreinigt werden könnten.

WO 89/09071 offenbart eine Spritze mit einem Luer-Lock-Anschluss mit einer drehbaren Gewindehülse, wobei an der Gewindehülse 4 radial nach außen gerichtete, voneinander wegweisende Stege angeordnet sind. Diese ermöglichen zwar eine bessere Kraftübertragung auf die Hülse, dennoch besteht, wenn die Hülse zwischen diesen Stegen ergriffen wird die Gefahr, dass die Finger unbeabsichtigt die Spitze des Luer-Konus berühren.

DE 10 2009 019 340 A1 offenbart eine Spritze mit einem Luer-Lock-Anschluss mit einer drehbaren Gewindehülse, wobei zwischen dem Luer-Konus und der Gewindehülse Klemmmittel zur Verdreh-Sicherung vorgesehen sind.

Im Hinblick auf diesen Stand der Technik ist es Aufgabe der Erfindung, eine Spritze, insbesondere eine medizinische Spritze mit einem Luer-Lock-Anschluss, welcher eine drehbare Gewindehülse aufweist, dahingehend zu verbessern, dass eine gute Handhabbarkeit mit gleichzeitig verringerter Gefahr von Verunreinigungen gegeben ist.

Diese Aufgabe wird durch eine Spritze mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Bei der erfindungsgemäßen Spritze handelt es sich insbesondere um eine medizinische Spritze, welche beispielsweise zur Aufnahme und Verabreichung von Arzneimitteln dienen kann. Die Spritze weist in bekannter Weise einen Spritzenzylinder auf, in welchem ein beweglicher Kolben angeordnet sein kann. An einem axialen Ende des Spritzenzylinders ist darüber hinaus in bekannter Weise ein Luer-Lock-Anschluss angeordnet. D. h. der Anschluss weist einen Luer-Konus und eine diesen umfänglich umgebende Hülse mit einem Innengewinde auf. Erfindungsgemäß ist dabei vorgesehen, dass die Gewindehülse drehbar ist. D. h. der Eingriff des Gewindes in der Gewindehülse mit dem an dem Luer-Konus zu befestigenden Gegenstück erfolgt durch Drehung der Gewindehülse relativ zu diesem Gegenstück. Das Gegenstück greift dabei mit einem korrespondierenden Gewinde oder Vorsprung in das Gewinde der Gewindehülse ein.

Erfindungsgemäß ist vorgesehen, dass die drehbare Gewindehülse eine radial nach außen gerichtete Auskragung aufweist. Die Auskragung bildet einen Griffbereich, an welchem die Gewindehülse zu ihrer Drehung ergriffen werden kann. Dadurch, dass die Auskragung einen Durchmesser aufweist, welcher gegenüber dem Durchmesser der Gewindehülse vergrößert ist, kann diese Auskragung gut ergriffen werden. Darüber hinaus bietet die Auskragung einen Griffbereich, welcher von der Spitze des Luer-Konus entfernt ist, da eine radiale Beabstandung gegeben ist. So ist die Gefahr der Berührung der Spitze des Luer-Konus beim Ergreifen der Gewindehülse minimiert. Darüber hinaus weist der so gebildete Griffbereich am Außenumfang der Auskragung wegen des größeren Durchmessers eine bessere Hebelwirkung bei Drehung der Gewindehülse auf, was dann von Vorteil ist, wenn die Gewindehülse, wie weiter unten beschrieben wird, zusätzlich mit Klemmmitteln versehen ist, welche die Gewindehülse nach ihrem Festziehen fixieren. Für eine solche Gewindehülse ist zum Festziehen eine größere Kraft erforderlich, die über den Griffbereich mit größerem Durchmesser, welcher von der Auskragung gebildet wird, einfacher aufgebracht werden kann.

Gemäß einer ersten bevorzugten Ausführungsform der Erfindung ist die radial nach außen gerichtete Auskragung an dem dem Spritzenzylinder zugewandten axialen Ende der Gewindehülse angeordnet. Diese Anordnung hat den Vorteil, dass die Auskragung in axialer Richtung möglichst weit von der Spitze des Luer-Konus entfernt ist. So wird die Gefahr der Berührung der Spitze des Luer-Konus beim Ergreifen der Gewindehülse weiter minimiert. Ferner kann so die Auskragung sich auch in axialer Richtung am Axialende des Spritzenzylinders abstützen, sodass eine größere Stabilität gegeben ist, und beispielsweise ein Abbrechen der Auskragung durch Druck in axialer Richtung verhindert werden kann.

Gemäß einer alternativen Ausführungsform ist es jedoch auch möglich, dass die radial nach außen gerichtete Auskragung von dem dem Spritzenzylinder zugewandten axialen Ende der Gewindehülse in axialer Richtung beabstandet ist. Dabei kann die Auskragung an dem dem Spritzenzylinder abgewandten Axialende der Gewindehülse angeordnet sein oder aber auch in einem axialen Mittelbereich zwischen den beiden Axialenden der Gewindehülse. Die axiale Beabstandung von dem Spritzenzylinder kann von Vorteil sein, um eine leichtere Drehbarkeit der Gewindehülse zu erreichen, da so die Auskragung der Gewindehülse einfacher ergriffen werden kann, ohne gleichzeitig den Spritzenzylinder festzuhalten. Wenn die Auskragung im axialen Mittelbereich der Gewindehülse angeordnet ist, kann ein guter Kompromiss zwischen leichter Drehbarkeit und axialem Abstand von der Spitze des Luer-Konus erzielt werden.

Die radial nach außen gerichtete Auskragung weist an ihrem radial äußeren Ende auf jeden Fall einen größeren Radius auf, als die Gewindehülse an ihrem Außenumfang. Bevorzugt ist der Radius der Auskragung von der Mittelachse des Spritzenzylinders zumindest so groß, dass er der Hälfte des Radius des Spritzenzylinders an dessen axialen Ende entspricht. Dieses axiale Ende ist das axiale Ende, welches dem Luer-Konus zugewandt ist. Mit diesem Radius wird sichergestellt, dass die Auskragung gut von außen ergriffen werden kann. Weiter bevorzugt entspricht der Radius der Auskragung von der Mittelachse des Spritzenzylinders zumindest zwei Dritteln des Radius des Spritzenzylinders an dessen axialen Ende. So kann die Auskragung noch besser ergriffen werden und hat an ihrem Außenumfang einen noch größeren radialen Abstand von dem Luer-Konus. Gemäß einer besonders bevorzugten Ausführungsform der Erfindung entspricht der Radius der Auskragung von der Mittelachse des Spritzenzylinders zumindest dem Radius des Spritzenzylinders an dessen axialen Ende. Bei dieser Ausführungsform lässt sich die Auskragung besonders gut ergreifen, da ihr Außenumfang zumindest dem Außenumfang des Spritzenzylinders an dessen dem Luer-Konus zugewandten Axialende entspricht.

Die radiale bzw. radial nach außen gerichtete Auskragung ist scheibenförmig ausgebildet. Die scheibenförmige Auskragung weist dabei weiter bevorzugt eine kreisförmige Form auf und ist konzentrisch zur Längs- bzw. Mittelachse des Spritzenzylinders angeordnet. Dies ist vorzugsweise auch die Achse, um welche die Gewindehülse drehbar ist. Durch die scheibenförmige Ausgestaltung kann die Auskragung von allen Umfangsseiten gut ergriffen werden. Darüber hinaus wird eine große Stabilität der Auskragung erzielt und der Boden des Spritzenzylinders, an welchem der Luer-Lock-Anschluss angeordnet ist, wird im Umfang des Luer-Anschlusses vorzugsweise vollständig von der Scheibe überdeckt. Dadurch wird gleichzeitig das Axialende des Spritzenzylinders vor Beschädigungen geschützt.

Besonders bevorzugt erstreckt sich die Auskragung scheibenförmig parallel zu einem den Luer-Konus umgebenden Boden des Spritzenzylinders. Dadurch fügt sich die scheibenförmige Auskragung harmonisch in die Gesamtgestaltung der Spritze ein. Gleichzeitig wird, wie oben beschrieben, der Boden des Spritzenzylinders überdeckt und vor Beschädigungen durch die Scheibe geschützt.

Gemäß einer ersten bevorzugten Ausführungsform ist die scheibenförmige Auskragung geschlossen ausgebildet. D. h. sie überdeckt den den Luer-Konus umgehenden Boden des Spritzenzylinders vollständig und weist vorzugsweise eine geschlossene Oberfläche auf. Dadurch wird verhindert, dass sich Verunreinigungen in der Scheibe festsetzen können.

Alternativ kann die scheibenförmige Auskragung auch so ausgebildet sein, dass sie eine oder mehrere Durchbrechungen aufweist. Solche Durchbrechungen können der Minimierung des Materialbedarfes dienen. Darüber hinaus können Durchbrechungen oder Strukturierungen in der scheibenförmigen Auskragung deren Gestaltung dienen. Beispielsweise könnten Pfeile, welche die Drehrichtung markieren als Durchbrechungen oder Ausnehmungen bzw. Vertiefungen in der scheibenförmigen Auskragung ausgebildet sein. Auch andere Markierungen, wie Hinweise auf die Inhaltsstoffe der Spritze, den Hersteller, etc. können in Form von Durchbrechungen oder Strukturierungen an der scheibenförmigen Auskragung angebracht werden.

Weiter bevorzugt hat die radiale Auskragung einen Radius, welcher größer als der Radius des Spritzenzylinders an dessen axialen Ende ist. Vorzugsweise steht die radiale Auskragung radial zumindest geringfügig über den Außenumfang des Bodens des Spritzenzylinders über. So kann die Auskragung an ihrem Außenumfang leicht ergriffen werden, ohne gleichzeitig den Spritzenzylinder festzuhalten. Ferner wird bei scheibenförmiger Ausgestaltung der Auskragung der Boden des Spritzenzylinders, das heißt der Boden an dem dem Luer-Konus zugewandten Ende, bevorzugt vollständig überdeckt und die scheibenförmige Auskragung kann gut am Außenumfang des Spritzenzylinders ergriffen werden.

Bevorzugt kann die Auskragung an ihrem Außenumfang strukturiert sein. Dies ermöglicht eine gute Griffigkeit und Kraftübertragung bei der Drehung.

Ferner kann die Auskragung in einer weiter bevorzugten Ausführungsform radial beabstandet von der Gewindehülse einen sich axial erstreckenden Vorsprung oder Kragen aufweisen. Der Vorsprung kann in Form einer Verdickung ausgebildet sein. Der Kragen kann bevorzugt hülsenförmig ausgebildet sein. Das heißt der Kragen weist eine zylindrische Gestalt auf und erstreckt sich bevorzugt parallel zur Längsachse des Spritzenzylinders bzw. des Luer-Konus. So kann z. B. bei scheibenförmiger Ausgestaltung der Auskragung die Scheibe am Außenumfang verdickt sein, sodass eine vergrößerte umfängliche Grifffläche geschaffen wird, welche es einfacher möglich macht, die Auskragung am Außenumfang zu ergreifen und zu drehen. Der axial erstreckende Vorsprung kann sich dabei vom Boden des Spritzenzylinders weg erstrecken, d. h. im Wesentlichen parallel zu der Längsachse des Luer-Konus. Alternativ oder zusätzlich kann er sich auch in der entgegengesetzten Richtung erstrecken, beispielsweise auch um ein gewisses Maß parallel zur Umfangswandung des Spritzenzylinders und diesen übergreifen. So wird eine vergrößerte Grifffläche am Außenumfang des Spritzenzylinders geschaffen. Wenn die Auskragung in axialer Richtung dem Spritzenzylinder zugewandten axialen Ende der Gewindehülse beabstandet ist, kann sich von der Auskragung bevorzugt ein Kragen in axialer Richtung zu dem Spritzenzylinder hin erstrecken. So wird der Freiraum zwischen der radialen Auskragung und dem Boden des Spritzenzylinders durch diesen Kragen im Wesentlichen umfänglich verschlossen. Der Kragen bildet dabei zum einen einen vergrößerten Griffbereich, verhindert zum anderen aber auch, dass Fremdkörper in den Freiraum zwischen Auskragung und Spritzenzylinder eindringen können. Darüber hinaus kann sich der Kragen bzw. Vorsprung in axialer Richtung am Boden des Spritzenzylinders abstützen, sodass die Auskragung bei axialem Druck am Spritzenzylinder abgestützt wird. Bevorzugt verbleibt zwischen dem Vorsprung bzw. dem Kragen und dem Boden des Spritzenzylinders ein geringer Abstand, um die leichte Drehbarkeit der Gewindehülse sicherzustellen. Bei axialem Druck auf die radiale Auskragung verringert sich dieser Spalt, bis im Extremfall der Kragen dann am Axialende des Spritzenzylinders zur Anlage kommen kann.

Besonders bevorzugt ist der Vorsprung oder Kragen am Außenumfang der radialen Auskragung angeordnet. Dabei schließt er sich weiter bevorzugt in axialer Verlängerung an den Außenumfang des Spritzenzylinders an, was zu einer optisch ansprechenden Gestaltung führt.

Die Gewindehülse ist drehbar an dem Spritzenzylinder, insbesondere dem Luer-Konus befestigt. Dazu kann die Gewindehülse zumindest einen radial nach innen gerichteten Vorsprung, insbesondere eine radial nach innen gerichtete Schulter aufweisen, welche in eine Nut am Fuß des Luer-Konus eingreift. So ist die Gewindehülse in axialer Richtung an den Luer-Konus gesichert, gleichzeitig aber drehbar. Um eine Sicherung der Luer-Lock-Verbindung zu erreichen, kann zwischen der Gewindehülse und dem Luer-Konus zumindest ein Reib- oder Klemmmittel angeordnet sein. Dadurch kann ein kraft- oder formschlüssiger Eingriff zwischen der Gewindehülse und dem Fuß des Luer-Konus erreicht werden, wenn die Gewindehülse mit einem auf dem Luer-Konus aufgesetzten Gegenstück verschraubt ist. Dadurch wird verhindert, dass die Gewindehülse sich wieder unbeabsichtigt löst. Diese Reibverbindung kann ausgestaltet sein, wie es in der deutschen Patentanmeldung 10 2009 019 340 offenbart ist.

Nachfolgend wird die Erfindung beispielhaft anhand der beigefügten Figuren beschrieben. In diesen zeigt:
- Fig. 1: eine perspektivische Gesamtansicht einer erfindungsgemäßen Spritze,
- Fig. 2: eine Schnittansicht des axialen Endes der Spritze gemäß Fig. 1,
- Fig. 3: eine perspektivische Gesamtansicht einer Spritze gemäß einer zweiten Ausführungsform der Erfindung,
- Fig. 4: eine Schnittansicht des axialen Endes der Spritze gemäß Fig. 3,
- Fig. 5: eine perspektivische Gesamtansicht einer Spritze gemäß einer dritten Ausführungsform der Erfindung,
- Fig. 6: eine Schnittansicht des axialen Endes der Spritze gemäß Fig. 5, und
- Fig. 7: eine Schnittansicht des axialen Endes einer erfindungsgemäßen Spritze gemäß einer vierten Ausführungsform der Erfindung.

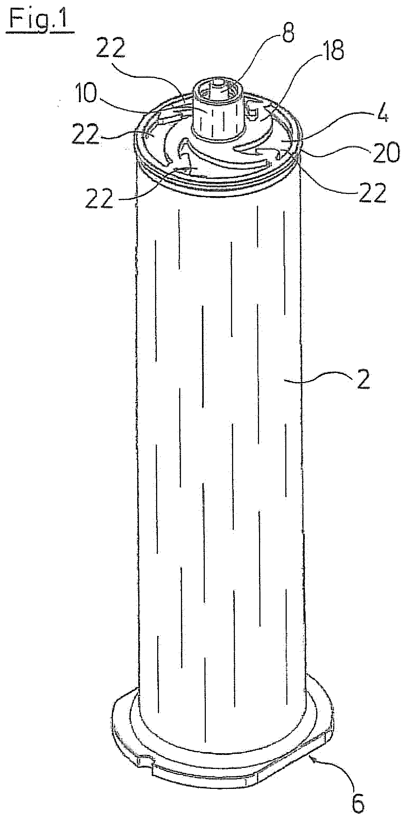

Die Figuren 1, 3 und 5 zeigen jeweils nur den Spritzenzylinder, der Kolben ist in den Figuren nicht erkennbar.

Die in Fig. 1 gezeigte Spritze gemäß einer ersten Ausführungsform der Erfindung weist in bekannter Weise einen Spritzenzylinder 2 auf, welcher an einem ersten, vorderen Axialende durch einen Boden 4 geschlossen ist und an dem entgegengesetzten zweiten Axialende 6 offen ausgebildet ist, wobei durch das zweite Axialende 6 in bekannter Weise ein Kolben in das Innere des Spritzzylinders 2 eingeführt werden kann. Der Boden 4 weist eine zentrale Öffnung auf, an welcher ein Luer-Konus 8 ausgebildet ist. Der Luer-Konus 8 weist in seinem Inneren eine sich in das Innere des Spritzenzylinders 2 erstreckende Öffnung auf und bildet die Ein- und Austrittsöffnung der Spritze. Der Luer-Anschluss ist bei der erfindungsgemäßen Spritze als Luer-Lock-Anschluss ausgebildet. So ist um den Luer-Konus 8 herum radial beabstandet eine Gewindehülse 10, welche an ihrem Innenumfang in bekannter Weise ein Innengewinde 12 und an ihrem dem Boden 4 zugewandten Fuß eine radial nach innen gerichtete Schulter 14 aufweist, welche in eine ringförmige Nut 16 am Fuß des Luer-Konus 8 eingreift. So ist die Gewindehülse 10 in axialer Richtung X an dem Luer-Konus 8 gesichert, aber um die Längsachse X relativ zu dem Luer-Konus 8 drehbar, um mit einem auf dem Luer-Konus 8 aufgesetzten Gegenstück, welches in den Freiraum zwischen Luer-Konus 8 und Gewindehülse 10 eingreift, zu dessen Sicherung verschraubt zu werden.

Erfindungsgemäß weist die Gewindehülse 10 eine radial nach außen gerichtete Auskragung 18 auf. Die Auskragung 18 ist im Wesentlichen scheibenförmig ausgebildet und erstreckt sich parallel zum Boden 4 des Spritzenzylinders 2. Dabei hat die scheibenförmige Auskragung 18 bezogen auf die Mittelachse X einen geringfügig größeren Durchmesser als der Spritzenzylinder 2 an seinem Boden 4. So steht die Auskragung 18 mit ihrem Rand nach außen über den Außenumfang des Spritzenzylinders 2 über und bildet so eine umfängliche Grifffläche 20, an welcher die Auskragung 18 und damit die Gewindehülse 10 ergriffen und gedreht werden kann. Dies hat den Vorteil, dass die Gewindehülse 10 nicht in einem Bereich angefasst werden muss, in welchem die Gefahr besteht, dass versehentlich die Spitze des Luer-Konus 8 berührt und dabei verunreinigt wird. Darüber hinaus wird so ein größerer Durchmesser zum Ergreifen der Gewindehülse 10 bereitgestellt, sodass die Gewindehülse 10 leichter gedreht werden kann. Ferner liegt die Grifffläche 20 radial außerhalb des Außenumfanges des Spritzenzylinders 2, sodass auch der Spritzenzylinder 2 das Ergreifen und Drehen der Gewindehülse 10 nicht stört.

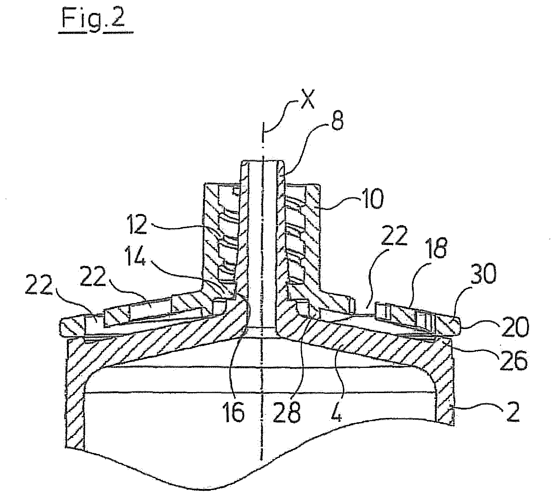

In dem in Figuren 1 und 2 gezeigten Beispiel ist die Auskragung 18 mit Durchbrechungen 22 versehen, welche hier so geformt sind, dass die verbleibenden Stege zwischen den Durchbrechungen 22 als Pfeile die Drehrichtung für die Gewindehülse 10 markieren.

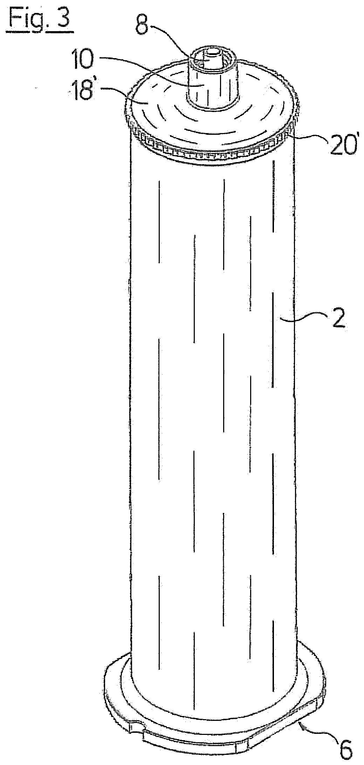

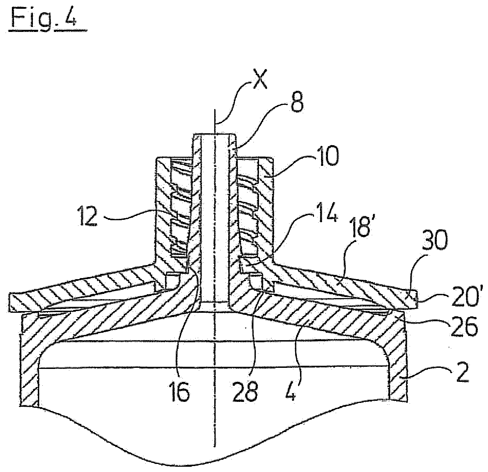

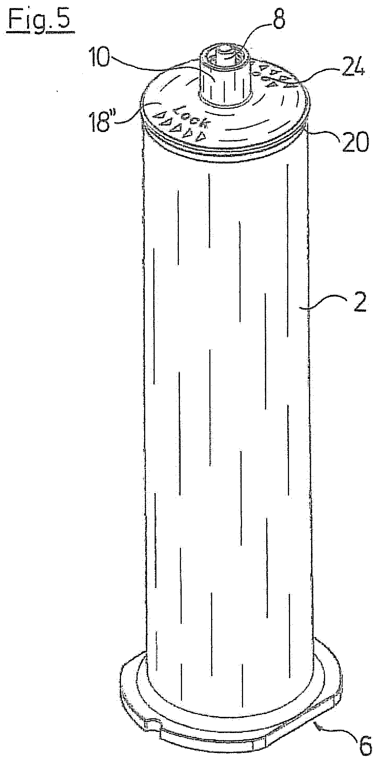

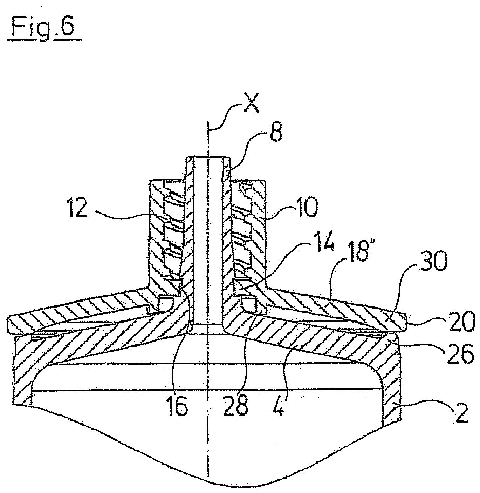

Die Figuren 3 und 4 zeigen ein zweites Ausführungsbeispiel der Erfindung, welches sich von dem anhand von Figuren 1 und 2 erläuterten Ausführungsbeispiel lediglich in der Gestaltung der Auskragung 18' unterscheidet. Alle übrigen Merkmale sind identisch. Bei dem in Fig. 3 und 4 gezeigten Beispiel ist die Auskragung 18' als geschlossene Scheibe mit geschlossenen Oberflächen ausgebildet. So überdeckt die Auskragung 18' den Boden 4 des Spritzenzylinders 2 vollständig. Ferner ist die Grifffläche 20' am Außenumfang der Auskragung 18' in dem hier gezeigten Beispiel geriffelt ausgebildet, sodass sie einen festeren Griff ermöglicht. Die dritte Ausführungsform, welche in den Fig. 5 und 6 gezeigt ist, unterscheidet sich von den zwei ersten Ausführungsformen darin, dass die scheibenförmig Auskragung 18" geschlossen ausgebildet ist, jedoch mit einer Beschriftung 24 an der Oberfläche versehen ist. Darüber hinaus ist die Grifffläche 20 bei dieser Ausführungsform wiederum glatt ausgebildet, könnte jedoch auch strukturiert bzw. geriffelt ausgebildet sein, wie in Fig. 3 gezeigt.

Bei allen drei Ausführungsbeispielen erstreckt sich die Auskragung 18, 18', 18" im Wesentlichen parallel zum Boden 4 am axialen Ende des Spritzenzylinders 2. Dabei liegt die Auskragung 18, 18', 18" jedoch nicht flächig auf dem Boden 4 auf, sondern wird beabstandet zu diesem geführt. Dazu sind Stege vorgesehen, nämlich ein Steg 26, welcher sich am Außenumfang des Bodens 4 in axialer Richtung X erstreckt. Ferner ist dies ein Steg 28, welcher sich im Bereich des Innenumfanges der Auskragung 18, 18', 18" im Wesentlichen im Erstreckungsbereich der Gewindehülse 10 in entgegengesetzter Richtung zu dieser axial von der Auskragung 18, 18', 18" wegerstreckt. Der Steg 28 liegt mit seinem freien Ende auf dem Boden 4 auf. Das freie Ende des Steges 26 liegt an der Auskragung 18, 18', 18" an. Dabei weist die Auskragung 18" im Bereich ihres Außenumfanges eine Verdickung 30 auf, welche auf dem Steg 26 zu liegen kommt. Auf diese Weise wird die Auskragung 18, 18', 18" in den übrigen Bereichen beabstandet von dem Boden 4 gehalten, wodurch sich die Reibung zwischen der Auskragung 18, 18', 18" und dem Boden 4 verringert. Die Verdickung 30 am Außenumfang der Auskragung 18, 18', 18" hat darüber hinaus den Vorteil, dass die Grifffläche 20, 20' breiter ausgebildet ist.

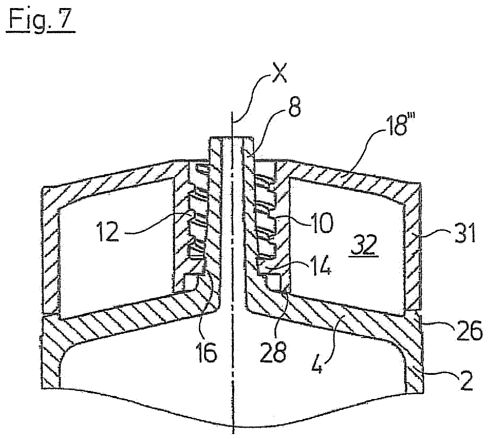

Bei der in Fig. 7 gezeigten vierten Ausführungsform der Erfindung ist im Unterschied zu der dritten Ausführungsform, welche in Fig. 6 gezeigt ist, die scheibenförmige Auskragung 18''' nicht an dem dem Spritzenzylinder 2 zugewandten axialen Ende der Gewindehülse 10 angeordnet, sondern am entgegengesetzten, dem Spritzenzylinder 2 abgewandten Axialende der Gewindehülse 10 angeordnet. Die scheibenförmige Auskragung 18''' ist auch hier, wie bei den vorangehenden Ausführungsformen, fest mit der Gewindehülse 10 verbunden, nämlich einstückig mit dieser ausgeformt. Die scheibenförmige Auskragung 18''' erstreckt sich im Wesentlichen parallel zum Boden 4 des Spritzenzylinders 2. Auch hier hat die scheibenförmige Auskragung 18''' einen Radius bzw. Durchmesser, welcher geringfügig größer als der äußere Radius des Spritzenzylinders 2 ist. Am Außenumfang der scheibenförmigen Auskragung 18''' ist ein zylindrischer Kragen 31 angeordnet, welcher sich von der Auskragung 18''' zu dem Spritzenzylinder 2 hin erstreckt. Der Kragen 31 weist einen Außendurchmesser auf, welcher im Wesentlichen dem Außendurchmesser des Spritzenzylinders 2 erstreckt, sodass er sich in axialer Richtung als axiale Verlängerung an diesen anschließt. Der zylindrische bzw. umfängliche Kragen 31 verschließt den Freiraum 32 zwischen der Auskragung 18''' und dem Boden 4 des Spritzenzylinders 2 außenumfänglich. Dadurch verhindert der Kragen 31, dass Fremdstoffe in den Freiraum 32 eindringen können und bietet gleichzeitig eine vergrößerte Grifffläche am Außenumfang, sodass die Auskragung 18''' noch leichter ergriffen und gedreht werden kann.

Ferner können bei allen vier Ausführungsformen zwischen der Schulter 14 und der Nut 16 Reib- oder Klemmmittel angeordnet sein, beispielsweise indem Teile der Schulter 14 oder der Nut 16 aufgeraut sind. Auch kann hier eine exzentrische Anordnung zwischen Schulter 14 und Nut 16 vorgesehen sein, um bei Drehung der Gewindehülse 10 bei eingesetztem Gegenstück eine Klemmung zwischen der Schulter 14 und der Nut 16 zu erreichen, um zu verhindern, dass die Gewindehülse 10 sich unbeabsichtigt löst.

### Bezugszeichenliste

- 2: - Spritzenzylinder
- 4: - Boden
- 6: - zweites Axialende
- 8: - Luer-Konus
- 10: - Gewindehülse
- 12: - Innengewinde
- 14: - Schulter
- 16: - Nut
- 18, 18', 18", 18''': - Auskragung
- 20, 20': - Grifffläche
- 22: - Durchbrechungen
- 24: - Beschriftung
- 26: - Steg
- 28: - Steg
- 30: - Verdickung
- 31: - Kragen
- 32: - Freiraum
- X: -Längs- bzw. Mittelachse

## Patentansprüche

1. Spritze mit einem Spritzenzylinder (2) und einem an einem axialen Ende des Spritzenzylinders (2) angeordneten Luer-Lock-Anschluss, welcher einen Luer-Konus (8) und eine diesen umgebende drehbare Gewindehülse (10) aufweist,
**dadurch gekennzeichnet, dass**
die drehbare Gewindehülse (10) des Luer-Lock-Anschlusses eine radial nach außen gerichtete scheibenförmig ausgebildete Auskragung (18, 18', 18", 18''') aufweist, welche einen Griffbereich (20, 20') bildet, an welchem die Gewindehülse (10) zu ihrer Drehung ergriffen werden kann.

2. Spritze nach Anspruch 1, **dadurch gekennzeichnet, dass** die radial nach außen gerichtete Auskragung (18, 18', 18") an dem dem Spritzenzylinder (2) zugewandten axialen Ende der Gewindehülse (10) angeordnet ist.

3. Spritze nach Anspruch 1, **dadurch gekennzeichnet, dass** die radial nach außen gerichtete Auskragung (18''') von dem dem Spritzenzylinder (2) zugewandten axialen Ende der Gewindehülse (10) in axialer Richtung beabstandet ist.

4. Spritze nach einem der Ansprüche 1 bis 3, **dadurch gekennzeichnet, dass** der Radius der Auskragung von der Mittelachse (X) des Spritzenzylinders (2) zumindest der Hälfte des Radius des Spritzenzylinders (2) an dessen axialen Ende entspricht.

5. Spritze nach einem der vorangehenden Ansprüche, **dadurch gekennzeichnet, dass** der Radius der Auskragung von der Mittelachse (X) des Spritzenzylinders (2) zumindest zwei Dritteln des Radius des Spritzenzylinders (2) an dessen axialen Ende entspricht.

6. Spritze nach einem der vorangehenden Ansprüche, **dadurch gekennzeichnet, dass** der Radius der Auskragung von der Mittelachse (X) des Spritzenzylinders (2) zumindest dem Radius des Spritzenzylinders (2) an dessen axialen Ende entspricht.

7. Spritze nach einem der vorangehenden Ansprüche, **dadurch gekennzeichnet, dass** die Auskragung (18, 18', 18") sich scheibenförmig parallel zu einem den Luer-Konus (8) umgebenden Boden (4) des Spritzenzylinders (2) erstreckt.

8. Spritze nach einem der vorangehenden Ansprüche, **dadurch gekennzeichnet, dass** die scheibenförmige Auskragung (18', 18", 18''') geschlossen ausgebildet ist.

9. Spritze nach einem der vorangehenden Ansprüche, **dadurch gekennzeichnet, dass** die scheibenförmige Auskragung (18) eine oder mehrere Durchbrechungen (22) aufweist.

10. Spritze nach einem der vorangehenden Ansprüche, **dadurch gekennzeichnet, dass** die radiale Auskragung (18, 18', 18", 18''') einen Radius hat, welcher größer als der Radius des Spritzenzylinders (2) an dessen axialen Ende ist.

11. Spritze nach einem der vorangehenden Ansprüche, **dadurch gekennzeichnet, dass** die Auskragung (18') an ihrem Außenumfang (20') strukturiert ist.

12. Spritze nach einem der vorangehenden Ansprüche, **dadurch gekennzeichnet, dass** die Auskragung (18, 18', 18", 18''') radial beabstandet von der Gewindehülse einen sich axial erstreckenden Vorsprung (30) oder Kragen (31) aufweist.

13. Spritze nach einem der vorangehenden Ansprüche, **dadurch gekennzeichnet, dass** die Gewindehülse (10) zumindest einen radial nach innen gerichteten Vorsprung (14) aufweist, welcher in eine Nut (16) am Fuß des Luer-Konus (8) eingreift.

14. Spritze nach einem der vorangehenden Ansprüche, **dadurch gekennzeichnet, dass** zwischen der Gewindehülse (10) und dem Luer-Konus (8) zumindest ein Reib- oder Klemmmittel angeordnet ist.

## Claims

1. A syringe with a syringe cylinder (2) and with a Luer-lock connection which is arranged at an axial end of the syringe cylinder (2) and which comprises a Luer cone (8) and a rotatable threaded sleeve (10) surrounding this, **characterised in that** the rotatable threaded sleeve (10) of the Luer-lock connection comprises a radially outwardly directed prominence (18, 18', 18", 18''') which is designed in a disc-like manner and which forms a grip region, on which the threaded sleeve (10) can be gripped for its rotation.

2. A syringe according to claim 1, **characterised in that** the radially outwardly directed prominence (18, 18', 18") is arranged at the axial end of the threaded sleeve (10) which is faces the syringe cylinder (2).

3. A syringe according to claim 1, **characterised in that** the radially outwardly directed prominence (18''') is distanced in the axial direction to the axial end of the threaded sleeve (10) which faces the syringe cylinder (2).

4. A syringe according to one of the claims 1 to 3, **characterised in that** the radius of the prominence from the middle axis (X) of the syringe cylinder (2) corresponds to at least half the radius of the syringe cylinder (2) at its axial end.

5. A syringe according to one of the preceding claims, **characterised in that** the radius of the prominence from the middle axis (X) of the syringe cylinder (2) corresponds to at least two thirds of the radius of the syringe cylinder (2) at its axial end.

6. A syringe according to one of the preceding claims, **characterised in that** the radius of the prominence from the middle axis (X) of the syringe cylinder (2) corresponds at least to the radius of the syringe cylinder (2) at its axial end.

7. A syringe according to one of the preceding claims, **characterised in that** the prominence (18, 18', 18") extends in a disc-like manner parallel to a base (4) of the syringe cylinder (2) which surrounds the Luer cone (8).

8. A syringe according to one of the preceding claims, **characterised in that** the disk-like prominence (18, 18', 18") is designed in a closed manner.

9. A syringe according to one of the preceding claims, **characterised in that** the disk-like prominence (18) comprises one or more openings (22).

10. A syringe according to one of the preceding claims, **characterised in that** the radial prominence (18, 18', 18", 18''') has a radius which is greater than the radius of the syringe cylinder (2) at its axial end.

11. A syringe according to one of the preceding claims, **characterised in that** the prominence (18') is structured on its outer periphery (20').

12. A syringe according to one of the preceding claims, **characterised in that** the prominence (18, 18', 18", 18'''), radially distanced to the threaded sleeve, comprises an axially extending projection (30) or collar (31).

13. A syringe according to one of the preceding claims, **characterised in that** the threaded sleeve (10) comprises at least one radially inwardly directed projection (14) which engages into a groove (16) on the foot of the Luer cone (8).

14. A syringe according to one of the preceding claims, **characterised in that** at least one friction means or clamping means is arranged between the threaded sleeve (10) and the Luer cone (8).

## Revendications

1. Seringue comportant un cylindre de seringue (2) et un raccord Luer-Lock disposé au niveau d'une extrémité axiale du cylindre de seringue (2), raccord qui présente un cône Luer (8) et une douille filetée tournante (10), entourant ce dernier, **caractérisée en ce que** la douille filetée tournante (10) du raccord Luer-Lock présente une saillie (18, 18', 18", 18'''), configurée en forme de disque et radialement dirigée vers l'extérieur, qui forme une zone de préhension (20, 20') au niveau de laquelle la douille filetée (10) peut être saisie pour assurer sa rotation.

2. Seringue selon la revendication 1, **caractérisée en ce que** la saillie (18, 18', 18") dirigée radialement vers l'extérieur est disposée au niveau de l'extrémité axiale, dirigée vers le cylindre de seringue (2), de la douille filetée (10).

3. Seringue selon la revendication 1, **caractérisée en ce que** la saillie (18''') dirigée radialement vers l'extérieur est située à une certaine distance, dans la direction axiale, de l'extrémité axiale de la douille filetée (10) dirigée vers le cylindre de seringue (2).

4. Seringue selon l'une des revendications 1 à 3, **caractérisée en ce que** le rayon de la saillie correspond, à partir de l'axe central (X) du cylindre de seringue (2), au moins à la moitié du rayon du cylindre de seringue (2) au niveau de son extrémité axiale.

5. Seringue selon l'une des revendications précédentes, **caractérisée en ce que** le rayon de la saillie, à partir de l'axe central (X) du cylindre de seringue (2), correspond au moins aux deux tiers du rayon du cylindre de seringue (2) au niveau de son extrémité axiale.

6. Seringue selon l'une des revendications précédentes, **caractérisée en ce que** le rayon de la saillie, à partir de l'axe central (X) du cylindre de seringue (2), correspond au moins au rayon du cylindre de seringue (2) au niveau de son extrémité axiale.

7. Seringue selon l'une des revendications précédentes, **caractérisée en ce que** la saillie (18, 18', 18") s'étend en forme de disque parallèlement à un fond (4), entourant le cône Luer (8), du cylindre de seringue (2).

8. Seringue selon l'une des revendications précédentes,**caractérisée en ce que** la saillie (18', 18", 18''') en forme de disque est configurée pleine.

9. Seringue selon l'une des revendications précédentes, **caractérisée en ce que** la saillie (18) en forme de disque présente une ou plusieurs ouvertures (22).

10. Seringue selon l'une des revendications précédentes, **caractérisée en ce que** la saillie radiale (18, 18', 18", 18''') présente un rayon qui est supérieur au rayon du cylindre de seringue (2) au niveau de son extrémité axiale.

11. Seringue selon l'une des revendications précédentes, **caractérisée en ce que** la saillie (18') est structurée sur sa périphérie extérieure (20').

12. Seringue selon l'une des revendications précédentes, **caractérisée en ce que** la saillie (18, 18', 18", 18''') présente, à une certaine distance axiale de la douille filetée, un épaulement (30) ou une collerette (31) s'étendant dans la direction axiale.

13. Seringue selon l'une des revendications précédentes, **caractérisée en ce que** la douille filetée (10) présente au moins un épaulement (14) dirigé radialement vers l'intérieur, qui entre en prise avec une rainure (16) aménagée au niveau du pied du cône Luer (8).

14. Seringue selon l'une des revendications précédentes, **caractérisée en ce qu'**au moins un moyen de friction ou de serrage est disposé entre la douille filetée (10) et le cône Luer (8).
